# EUROPÄISCHE PATENTSCHRIFT

(11) **EP 2 978 885 B1**
(45) Veröffentlichungstag und Bekanntmachung des Hinweises auf die Patenterteilung: **13.05.2020**
(21) Anmeldenummer: 14718906.2
(22) Anmeldetag: 05.03.2014
(51) Int. Cl.: D04H 1/407, A61F 7/02, A61F 7/10, A61F 13/00

(54) **3D-VLIES**
3D-NONWOVEN
NON-TISSÉ TRIDIMENSIONNEL

(30) Priorität: 26.03.2013 DE 102013005130
(43) Veröffentlichungstag der Anmeldung: 03.02.2016
(73) Patentinhaber: pervormance international GmbH, 89075 Ulm (DE)
(72) Erfinder: RENNER, Gabriele, 89275 Elchingen (DE)
(74) Vertreter: Frick, Robert
(86) Internationale Anmeldenummer: PCT/EP2014/000550
(87) Internationale Veröffentlichungsnummer: WO 2014/154319

(56) Entgegenhaltungen:
- DE-A1-102005 041 117
- DE-A1-102007 036 496
- DE-U1-202010 012 517
- US-A- 5 031 418
- US-A1- 2005 118 383
- US-A1- 2008 268 216

## Beschreibung

Die Erfindung betrifft die nicht-therapeutische Verwendung eines dreidimensionalen Vliesmaterials, das die Temperatur auf spezifischen Körperteilen in geringem Maße reduziert und dadurch direkt durch Entzug von Energie an diesen Körperteilen und/oder indirekt durch spezielle Mechanismen des Fettstoffwechsels eine fettabbauende Wirkung hervorruft und damit Fett reduzieren kann. Zudem kann durch die Temperaturreduktion auch ein hautstraffender Effekt hervorgerufen werden. Die Erfindung zeichnet sich dadurch aus, dass die Körperstellen definiert werden und in Form der Kombination des 3D- Vliesmaterials mit lipolytischen, fettabbauenden und/oder hautstraffenden Wirkstoffen der lipolytische, fettabbauende bzw. hautstraffende Effekt weiter verstärkt werden kann.

### Beschreibung:

Der menschliche Körper besitzt eine Körperkerntemperatur von 36,8 Grad Celcius und auf seiner Körperoberfläche eine Temperaturverteilung von ca. 28-36 Grad Celcius. Bei höheren oder niedrigeren Temperaturen beginnt der Körper durch die körpereigene Thermoregulation den Körper auf den optimalen Temperaturbereichen insbesondere auf der optimalen Körperkerntemperatur zu halten, da nur in diesem Temperaturbereich alle Prozesse im Körper optimal funktionieren. Hierzu wendet der Körper enorm viel Energie auf, da dieser Prozess lebensnotwendig ist. Bereits bei Temperaturen von 38°C oder 39°C ("Fieber")ist der Körper geschwächt und Körperkerntemperaturen von über 40°C können lebensbedrohlich sein. Ebenso Temperaturabweichungen nach unten wie z.B. 33-35°C haben deutliche Effekte auf körpereigene Prozesse und können zu einer Schwächung des Körpers und gesundheitlichen Problemen führen.

Die Absenkung der Hauttemperatur führt nicht in demselben Maße zu diesen extremen Reaktionen. Dennoch kann bei längerer Anwendung von sehr warmen oder sehr kalten Temperaturen auf der Haut eine Reaktion auch auf den Körperkern erfolgen. Das Hautsystem kann aber zumindest für kürzere Zeiträume dazu verwendet werden dem Körper Energie zu entziehen ohne gesundheitlich kritische Reaktionen des Körpers hervorzurufen. Daher sollten keine zu tiefen Temperaturen zur Kühlung verwendet werden und wenn dann diese möglichst nur unter Kontrolle verwendet werden.

Aufgrund der Energie, die durch die körpereigene Thermoregulation benötigt wird, wird dem Körper Energie entzogen und dadurch unter anderem Fettgewebe abgebaut um die notwendige Energie zu liefern. Zudem wurde in Studien gefunden, dass gerade durch lokale Kälte dem Körper an der gekühlten Stelle Energie entzogen wird und dies zu einem regionalen Abbau von Fettgewebe im Körper führt. Vor allem sogenanntes braunes Fett ist dazu geeignet bei kühleren Temperaturen von unter 20 Grad Celcius so zu kommunizieren, dass es zum Fettabbau kommt. Forschungen haben ergeben, dass ein Mangel an braunem Fettgewebe zu verstärkter Stimulation der weißen Fettdepots führt welche durch Botenstoffe des sympathischen Nervensystems erfolgt. Es wurde gezeigt, dass das braune Fettgewebe mit dem weißen Fettgewebe kommuniziert um die Körpertemperatur zu regulieren. Umgekehrt kann durch die Aktivierung von braunem Fettgewebe durch Kühlung der Abbau von weißem Fettgewebe forciert werden.

Somit können durch Kühlung der Haut zentrale und regionale Effekte auf den Fettabbau bzw. die Fettverbrennung hervorgerufen werden, die in dieser Schrift auch als lipolytische Effekte bezeichnet werden.

Hierbei wurde gefunden, dass die Temperaturreduktion auf der Haut nur eine leichte Abkühlung von ca. 24-28 °C Hauttemperatur benötigt und eine Kühlung mit einem Material, das 15-20°C bereitstellt ausreichend ist. Allerdings reagiert jeder Menschetwas anders auf Temperaturen so dass eine individuelle Anpassung und Flexibilität der Temperatureinstellung sinnvoll ist.

Da der Anstieg von Adipositas und Folgeerkrankungen wie z.B. Diabetes und Herz-Kreislauferkrankungen nicht nur zu einem gesundheitlichen Problem des Einzelnen sondern durch den enormen Anstieg dieser Problematik auch zu einer enormen Belastung des Gesundheitswesens geworden ist, ist es sinnvoll neben bekannten Maßnahmen neue Methoden des Fettabbaus am Körper voranzubringen. Zudem ist bekannt, dass auch bei sonst schlanken Personen das Herzinfarktrisiko mit stärkerem Bauchfett erhöht ist, so dass auch dieser Risikofaktor minimiert werden könnte. Des weiteren können auch Schönheitsaspekte, die Fettdepots an ungewünschten Stellen reduziert sehen wollen, hier profitieren bis hin zu Sportarten wie Bodybuilding bei denen jedes Fettdepot, das die definierte Ansicht der Muskulatur beeinträchtigt, stört.

Diese Methoden müssen vor allem bei den zuerst genannten Personenkreisen schnell und einfach und am besten mobil und vom Betroffenen selbst auch zuhause durchführbar sein. Die Anwendung muss zudem ohne großen Aufwand oder Nebenerscheinungen funktionieren und angenehm sein.

Wie man aus den Studien zu Diäten oder Bewegungsaktivitäten weiß, ist der Erfolg stark mit der Akzeptanz des Verwenders gekoppelt. Eine unangenehme, aufwändige, zeitintensive oder unangenehme Maßnahme wird entweder gar nicht oder nicht konsequent durchgeführt und führt damit auch nicht zum Erfolg. Desweiteren handelt es sich bei diesen Erkrankungen um eine "Volkskrankheit" wodurch die Kosten für etwaige Maßnahmen nicht zu hoch sein dürfen, da diese sonst nicht von Krankenkassen bzw. vom Gesundheitssystem nicht getragen werden können.

Im Stand der Technik sind bereits einige Kühlmethoden bekannt, die jedoch einige Nachteile haben:
Ein sehr aufwändiges Verfahren stellen Kühlkammern dar, bei denen man sich einige Minuten in auf unter 100-zu 150°C herunter gekühlten Räumen oder großen Behältern aufhält. Diese Kühlkammern sind jedoch aufgrund des Preises und des Energie- und Platzbedarfs nicht flächendeckend vorhanden. Zudem sind diese Minustemperaturen sehr unangenehm und müssen streng unter Aufsicht erfolgen. Studien zu diesen Kühlräumen und ihrer Wirkung auf den Fettabbau fehlen jedoch bislang.

Zum weiteren gibt es Apparaturen, die den zu kühlenden Bereich, der die Fettdepots besitzt, zwischen Platten einquetscht (Druckaufbau) und diese Platten zudem durch diese Apparatur gekühlt werden. Durch den Druck und die Kühlung werden laut dieser Anordnung die Fettzellen zum Platzen gebracht und das zerstörte Fettgewebe in den folgenden 4-8 Wochen abtransportiert. Die Anwendung muss jedoch bei einem dafür geschulten Personal unter Aufsicht durchgeführt werden. Die Anwendung ist nicht mobil so dass die Zeit hierfür aufgebracht werden muss und nicht alle Körperteile können in dieser Form zwischen den Platten eingebracht werden so dass die Methode keine Ganzkörpermethode darstellt und somit nur für einige Körperteile einsetzbar ist. Zudem entstehen durch Einsatz der sehr aufwändigen Apparatur, der Energiequelle und das Personal entsprechende Kosten.

Ein weiteres Produkt zur Kühlung ist ein Baumwollanzug bestehend aus einem sehr leichten und dünnen Baumwollgewebe ähnlich eines Mullbindenmaterials, das in einer Flüssigkeit aus Alkohol, Koffein, Carnitin und Coenzym A getränkt wird. Dieser Anzug wird dann angezogen und soll zum Fettabbau dienen. Der Anzug ist durch die Flüssigkeit natürlich nass und unangenehm, Erkältungsgefahr durch die nasskalte Feuchtigkeit ist gegeben und zudem kann das Material nicht gewaschen bzw. längere Zeit wiederverwendet werden. Dies macht eine Anwendung über längere Zeit teuer.

Eine weitere Kühlanordnung im Stand der Technik ist durch ein PCM System (PCM=Phase change material) gegeben, in dem Salz, die bei 14°C schmilzt verwendet wird, das in Plastik eingeschweißt in Westen eingebracht wird. Diese Salze schmelzen und geben dabei einen Kühleffekt ab, der zum Fettabbau dienen soll. Da die Zeit des Schmelzens mit ca. 60-90 min. begrenzt ist, wird durch eine Aufbewahrung und Regenerierung im Kühl- oder Eisschrank eine entsprechend aufwändige Logistik mit entsprechenden Kosten benötigt. Die Weste hat durch den Einsatz der Salzpakete ein deutliches Gewicht - zudem ist die Kühltemperatur von dauerhaften 14°C relativ kalt und wird nicht von jedem Anwender vertragen. Die Salzpakete in der Weste sind nicht nur schwer sondern auch hart auf der Haut und nicht anschmiegsam. Zudem entsteht durch die Kühlung Kondenswasser, das die Anwender nass und feucht werden lässt.

Somit haben alle bislang verfügbaren Kühlanordnungen deutliche Nachteile. Das Ziel der vorliegenden Erfindung soll die Nachteile der bisherigen Anordnungen überwinden und eine schnelle, einfache, trockene Kühlanordnung mit dem Ziel einer hohen Akzeptanz beim Verwender und einer geringen Kostenstruktur bereitstellen.

Vliesmaterialien, die mit einem textilen oder nicht-textilen Material umschlossen werden und zur Kühlung eines Oberflächenabschnitts des menschlichen Körpers grundsätzlich eingesetzt werden könnten, sind beispielsweise der DE 10 2007 036 496 A1 oder der DE 20 2010 012 517 U1 offenbart.

Die vorliegende Erfindung schlägt eine nicht-therapeutische Verwendung eines dreidimensionalen Vliesmaterials gemäß der Definition des Anspruchs 1 vor. Das Vliesmaterial besitzt eine dreidimensionale unregelmäßige Netzstruktur, die Superabsorber auf bzw. an den netzartig verflochtenen Vliesfasern besitzt. Die Flüssigkeitsbindung findet an den Fasern und deren Verknüpfungspunkten statt, was die Bindung der Flüssigkeit erhöht und die Oberfläche für die Bindung der Flüssigkeit vergrößert. Dadurch ist die Flüssigkeit fest gebunden und kann nicht durch Druck entweichen. Die Oberfläche ist damit trocken und der Körper wird bei der Verwendung nicht nass. Durch Verwendung einer textilen oder nicht textilen Hülle um das Vliesmaterial, die entweder ganz oder mindestens an der Oberseite vom Körper weg zeigenden Seite flüssigkeitsdurchlässig und flüssigkeitsdampfdurchlässig ist, kann auch ein entsprechendes Design der Erfindung ermöglicht werden um die Akzeptanz des Verwenders zu erhöhen. Das Material ist waschbar und somit immer wieder verwendbar, was Kosten spart.

Das erhöht die Compliance der Methode im Vergleich zum Stand der Technik, welche einen flächigen Baumwollstoff mit einer mullbindenähnlichen Struktur verwendet, die nass ist und tropft und reduziert die möglichen Nebenerscheinungen, die durch das nasse Material entstehen könnten deutlich. Die Wiederverwendbarkeit ist ebenfalls ein Vorteil zum Stand der Technik, da das mullbindenähnliche Material zwar einige Male verwendet werden kann, jedoch nicht waschbar ist und dann aus hygienischen Gründen entsorgt werden muss und ein neues Produkt verwendet werden muss, das wieder Kosten produziert.

Die Kühlung im erfindungsgemäßen dreidimensionalen Vliesmaterial wird durch Verdunstung der Flüssigkeit gewährleistet, die aufgrund der Dreidimensionalität und damit vorhandenen großen Oberfläche der Flüssigkeit in dem Vliesmaterial entsteht, das die Flüssigkeit in hohem Maße wieder abgibt und dadurch Temperaturen von 15-20°C erzeugt, die bei Verwendung auf der Haut zu Hauttemperaturen von 24-28°C führt und damit einen lipolytischen und damit fettabbauenden Effekt bewirkt.

Ziel ist es eine schnelle, einfache, mobile und angenehme Kühlanordnung zu bieten, die von den betroffenen Personen gerne und selbst organisatorisch immer wieder verwendet werden kann.

Um eine Kontrolle des Fettabbaus zu ermöglichen sowohl der Kühlwirkung als auch der lipolytischen Effekte, wurde diese Kontrolle bei der erfindungsgemäßen Aufgabe mitentwickelt.

Um die Kontrolle der Kühleffizienz zu gewährleisten, wird die Kühltemperatur gemessen. Dies kann in Form eines integrierten Thermometers, einer Vorrichtung mit Thermofarben- oder kristallen oder anderweitigen bekannten Temperaturmessvorrichtungen erfolgen.

Um die Kontrolle des Gewichtsverlustes zu erkennen, werden entweder an das Kühlsystem oder separat aber in Kombination damit Messvorrichtungen in Form von Waagen, Fettmessgeräten, Umfangmessgeräten, etc. verwendet, die durch automatische Übertragung oder durch Eingabe die Effizienz der Kühlvorrichtung in Form eines dokumentierten Ergebnisses erscheinen lässt. Dies kann ein Monitor sein oder ein PC, Handheld, Mobiltelefon, Mp3Player, oder anderes stationäres oder mobiles Endgerät sein. Die Ergebnisse können entweder zur Selbstkontrolle oder aber durch Übertragung an einen Dritten zur Kontrolle beim Arzt, Ernährungsberater oder an eine Plattform im Internet versendet werden, die eine Auswertung vornimmt und ein Feedback mit weiteren Verbesserungsvorschlägen anbietet um die Effizienz zu erhöhen. Hierdurch kann bei entsprechenden Erfolgsmeldungen und motivatorischen Elementen die Akzeptanz und damit Compliance verbessert werden. Dies kann auch spielerisch mit Apps oder anderen Programmen im stationären oder mobilen Endgerät, Intra- oder Internet bzw. anderen Kommunikationseinrichtungen geschehen.

Ziel ist es die Betroffenen bei der Fettabnahme zu unterstützen und die Compliance zu erhöhen. Wie zahlreiche Studien zeigen ist die Akzeptanz und das Durchhalten der Maßnahmen zum Fettabbau der wichtigste Faktor zum Erfolg.

Selbstverständlich kann die Kühlanordnung auch in Kombination mit Sport, Bewegung und oder Ernährungsumstellung bzw. Diäten verwendet werden um einen schnelleren Erfolg durch synergistische Effekte der einzelnen Methoden zu erzielen. Gerade hier ist der mobile Einsatz auch zu Hause von Vorteil.

Auch hier ist eine Kombination all dieser Methoden über neue Medien sinnvoll und wird durch die erfindungsgemäße Kühlanordnung unterstützt.

Wichtig ist außerdem die Anordnung des Kühlvlies am Körper durch ein sogenanntes Bodymapping in dem die Regionen festgelegt werden, die gekühlt werden sollen. Dabei ist es wichtig Bereiche die nicht an Volumen verlieren sollen wie z.B. der Brustbereich bei Frauen auszusparen. Dasselbe gilt für kälteempfindliche Bereiche wie z.B. der Bereich der Nieren und der Harnwege bzw. ggfs. der Genitalien.

Zudem ist es sinnvoll in einer weiteren Ausgestaltung der Erfindung die Bereiche in die Kühlbereiche einzubinden, die sich dadurch auszeichnen, dass sie Bereiche darstellen, die braunes Fett beinhalten um neben der Energieleistung die Kommunikation des Fettstoffwechsels zu verstärken und dadurch zusätzliche Effekte auf den Fettabbau zu gewährleisten. Hierzu zählen neben den Schulterblättern der Hals und die Wirbelsäule.

Eine Kompression der betroffenen Körperteile durch elastische, enge, die Körperteile komprimierende Stoffe kann sich ebenfalls positiv auf die Wirkung auswirken. Eine erfindungsgemäße Ausgestaltung wäre auch ein Modell in Form von Shapingunterwäsche, die an definierten Flächen bzw.- Körperstellen das erfindungsgemäße Vliesmaterial fest oder lösbar beinhaltet. Aber auch Wrappings und Kompressionskleidung oder komprimierende oder einfache Bandagen an bestimmten Körperteilen sind Gegenstand der Erfindung.

Die mit dem erfindungsgemäßen Vliesmaterial vollflächig oder teilweise ausgestatteten Kleidungsstücke oder Pads können verschiedenen Körperformen entsprechen oder nur für bestimmte Körperstellen bestimmt sein. Es können damit Kleidung wie Oberteile mit oder ohne Ärmel, kurz oder langärmelig, Westen, Shirts, Jacken, Kleider, Anzüge, Overalls, Shapingwäsche- und Kleidung, Kompressionsbekleidung- oder Accessoires, Röcke, Unterwäsche, Hosen, Leggings, Chaps oder Strümpfe ausgestattet werden. Ebenso kann das Vliesmaterial in Form von Pads fest oder lösbar in oder an Kleidung eingebracht werden.

Dabei kann das erfindungsgemäße Vlies fest oder lösbar in die Kleidung integriert sein. Es können mit Verschlüssen verbundene oder in Taschen eingebrachte Pads die das Vliesmaterial enthalten verwendet werden oder das Vliesmaterial kann vollflächig oder an bestimmten Stellen in die Kleidung eingenäht, eingeschweißt, eingesteppt oder anderweitig fest verbunden sein.

In unseren Untersuchungen wurde gefunden, dass durch die Kühlung entsprechender Körperteile bei einer täglichen bzw. alle zweitägigen Verwendung der Kühlanordnung innerhalb von 2 bzw. 4 Wochen z.B. eine Umfangreduktion im Bauchbereich von 2-4 cm erreicht wurde was einer halben bis einer Konfektionsgröße entspricht. In Einzelfällen wurden in 4 Wochen und länger auch eine Umfangreduktion von 4-8 mm gefunden was einer Reduktion von 2 Konfektionsgrößen entspricht. Gerade die verschiedenen Wirkmechanismen der regionalen Kühlung zeigen sich in den Ergebnissen.

**Tabelle Ergebnisse:**

| | | | |
|---|---|---|---|
| Bauchumfang bei täglicher Kühlung von mindestens 30 min. mit dem 3D Vlies, Wasser und | | | |
| in Kombination mit 5% Koffein und tägliche bzw. alle 2 Tage Kühlung. | | | |

| | -----------gemessener Bauchumfang in cm------------- | | |
|---|---|---|---|
| Zeit | Proband A | Proband B | Proband C |
| Tag 1 | 89 | 91 | 101 |
| Tag 2 | 89 | | 101 |
| Tag 3 | 88 | 91 | 100,5 |
| Tag 4 | 88 | | 100,5 |
| Tag 5 | 87,5 | 90,5 | 100,5 |
| Tag 6 | 87,5 | 90,5 | 100,5 |
| Tag 7 | 87 | | |
| Tag 8 | | 91 | 100 |
| Tag 9 | 87 | | 100 |
| Tag 10 | 87 | 90,5 | 100 |
| Tag 11 | 87 | | |
| Tag 12 | | 90,5 | 99,5 |
| Tag 13 | 86,5 | 90 | 99,5 |
| Tag 14 | 86,5 | 90 | 99,5 |
| Tag 15 | 86,5 | 90 | 99,5 |
| Tag 16 | | | 99 |
| Tag 17 | 86 | 89,5 | 99 |
| Tag 18 | | 89,5 | 99 |
| Tag 19 | 86,5 | | 99 |
| Tag 20 | | 90 | |
| Tag 21 | 86 | | 99,5 |
| Tag 22 | | 89,5 | 99,5 |
| Tag 23 | 86 | 89,5 | 99 |
| Tag 24 | | | |
| Tag 25 | 85,5 | 89 | 99 |
| Tag 26 | 85,5 | 89 | 98,5 |
| Tag 27 | 85 | 88,5 | 98,5 |
| Tag 28 | 85 | 88,5 | 98,5 |

Zudem wurde in den durchgeführten Studien gefunden, dass die Kühlung auch positive Effekte auf die Hautstruktur und das Bindegewebe hatte, was sich in einer Reduktion von Falten, einer gleichmäßigerer Hautstruktur und verminderter Cellulitis zeigte. Da durch die verminderten Fettdepots die Hautstruktur glatter wird ist auch nachgewiesen, dass durch Kälte z.B. die Durchblutung angeregt wird und die Kollagenproduktion erhöht wird. Somit können neben hautstraffenden auch faltenreduzierende Effekte erzielt werden. Der hautstraffende Effekt kann damit auch gegen die Erscheinungen von Zellulitis positiv wirken.

Die Verwendung des Kühlvlies erfolgt dadurch, dass das Kühlvlies mit einer wasserdurchlässigen Hülle in ein Bekleidungsoberteil und /oder ein Bekleidungsunterteil eingebracht wird, das am Körper anliegt. Dies kann durch direktes Einbringen des Kühlvlies in das Bekleidungsstück oder durch Pads, die in Taschen oder anderweitig in oder an das Bekleidungsstück angebracht sind, erfolgen.

Das Kühlvlies wird entweder mit dem Bekleidungsstück oder die Kühlvliespads vor An- oder Einbringen in das Bekleidungsstück mit einer Flüssigkeit wie z.B. Wasser getränkt, begossen oder besprüht so dass ca. 1 ml Flüssigkeit pro cm2 im Vlies enthalten sind. Wenn mehr Flüssigkeit verwendet wird, wird diese auch noch in hohem Masse gebunden. Das Produkt wird dadurch nur schwerer, die Funktion bleibt aber erhalten. Wenn zu viel Flüssigkeit verwendet wird, kann das Kühlvlies nach der Flüssigkeitszugabe einfach leicht mechanisch ausgedrückt werden und wenn notwendig die Hülle durch Abtrocknen mit einem Tuch oder durch andere Methoden getrocknet werden, so dass kein Wasser mehr in Form von Tropfen aus dem Kühlvlies entweicht. Sollte eine Hülle verwendet werden, die ein schnelles Trocknen nicht gewährleistet, kann der Artikel mit Wärme (Heizung, Fön, oder ähnliches) oberflächlich getrocknet werden. Die Flüssigkeit bleibt dennoch zu über 90% in dem Kühlvlies fest gebunden und kann durch Druck nicht weiter entweichen. In der erfindungsgemäßen Vorrichtung ist der Artikel dann einsatzbereit und kann zur Kühlung verwendet werden. Der Körper selbst wird durch die enthaltene Flüssigkeit nicht nass, was zu einer erhöhten Akzeptanz beim Verwender führt. Durch die Bindung der Flüssigkeit in der dreidimensionalen Netzstruktur des Vlies ist die Oberfläche sehr vergrößert und damit die Flüssigkeitsoberfläche auch stark vergrößert was zu einer erhöhten Verdunstungsleistung führt und somit zu einer stärkeren Kühlung als bei einer einfachen flächigen Stoffstruktur. Zudem nimmt diese vergrößerte Oberfläche schneller und besser Flüssigkeit auf und bindet diese dadurch und durch den in der dreidimensionalen Vliesstruktur auf den vernetzten Fasern gebundenen Superabsorber wird die Flüssigkeit fest gebunden, so dass diese nicht durch Druck entweicht und den Körper somit trocken hält. Dies verbessert die Trageeigenschaften enorm und bietet deutliche Vorteile in der Anwendung.

Etwa 5-10 Minuten nach dem Anziehen oder auflegen des Kleidungsstücks auf die Haut des Verwenders wird es kurz abgenommen und belüftet und mit der bislang äußeren Seite nach innen zur Haut wieder angezogen bzw. wieder aufgelegt. Dieser Wechsel erzielt eine höhere Kühlwirkung, da dadurch die vorhandene Wärme des Körpers regional vom Kühlvlies aufgenommen wird und abtransportiert wird. Die andere Seite des Kühlvlies ist deutlich kühler und kann dem Gewebe weitere Wärme entziehen wodurch die Zieltemperatur von 24-29°C auf der Haut schnell und andauernd erreicht wird. Dieser Vorgang wird wiederholt sobald das Kühlvlies auf der Haut zugewandten Seite sich wieder deutlich erwärmt.

Dies erfolgt also immer dann wenn die Temperatur an der Innenseite bzw. auf der Haut sich erwärmen sollte. Durch diesen Vorgang wird der Haut bzw. dem Körper Energie entzogen und der fettabbauende Prozess in Gang gesetzt.

Bei Betroffenen bei denen der Kühleffekt alleine durch Verdunstungskälte nicht ausreicht, kann der Effekt durch Vorkühlen des Materials im Kühlschrank, in einer Kühlbox oder einem anderen Kühlbehälter oder durch das Einbringen von handelsüblichen Eis- oder gefrorenen Gelpacks oder aktiven PCM Pads auf der Außenseite des Vlies verstärkt werden. Das Eis-,Gel- bzw. PCM Material soll dabei nicht in direkten Kontakt mit der Haut kommen um die diskutierten negativen Effekte zu verhindern. Die Vorkühlung soll in keinem Fall zu niedrigeren Temperaturen als 10°C führen um gesundheitliche Schäden der Kühlung an der Haut bzw. des Körpers (wie z.B. des Immunsystems) zu verhindern. Das dreidimensionale Vliesmaterial nimmt die Kälte aus dem außen angebrachten Eis- oder Gel- bzw. PCM-pack aufgrund seiner großen Oberfläche schnell auf und gibt diese in abgemilderter Form an die Haut weiter. Durch Einschubmöglichkeiten in Taschen oder lösbar angebracht z.B. mit Klettverschluß können diese Kühlverstärker auch nur an bestimmten Stellenverwendet oder nach einiger Zeit wieder abgetrennt oder herausgenommen werden.

Auch eine kurze feste Massage der betroffenen Körperteile direkt nach der Kühlung hat sich als vorteilhaft erwiesen, da durch den mechanischen Druck die durch die Kühlung und falls verwendet die lipolytisch wirkenden Wirkstoffe die angeschlagenen Fettzellen weiter zerstört werden. Ein Abtransport dieser Zellen wird beschleunigt. Auch eine Lymphdrainage bzw. diesem entsprechende Methoden erleichtern den Abtransport der Fettzellen zusätzlich.

### Beschreibung der Ansichten der Zeichnung

In der Zeichnung ist die Erfindung schematisch dargestellt. Es zeigt:

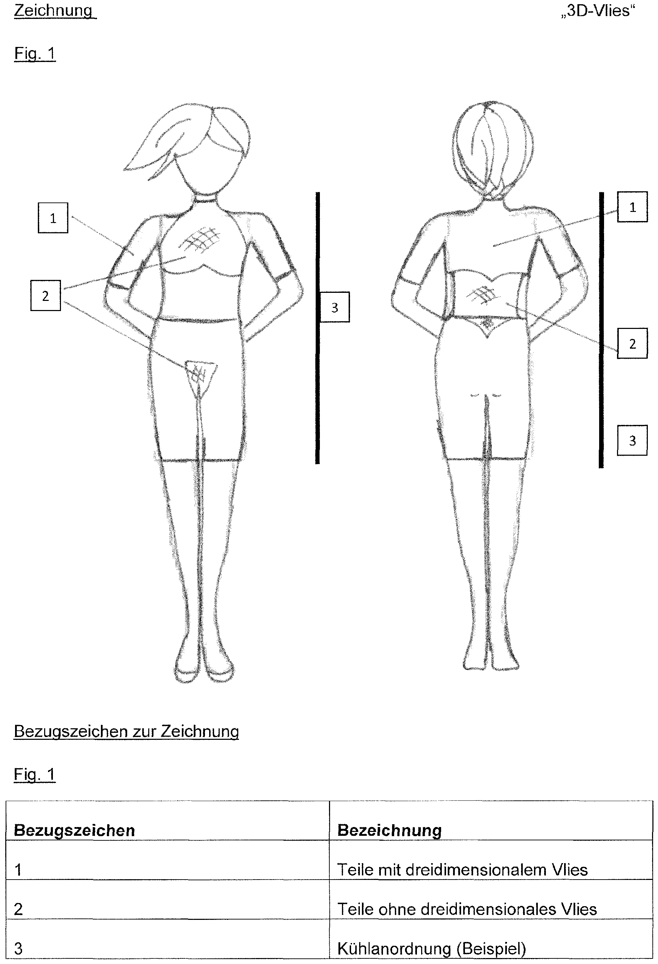

- Fig. 1: eine Vorder- und Rückenansicht von einer Variante einer erfindungsgemäßen Kühlanordnung (3) in Form eines Bekleidungsstückes bestehend aus Anteilen mit dreidimensionalem Kühlvlies (1) und mit Anteilen ohne dreidimensionales Kühlvlies (2), da diese Bereiche im Sinne des Bodymapping nicht gekühlt werden sollen, da diese Bereiche entweder besonders kälteempfindlich sind wie z.B. die Nieren oder Bereiche der Harnwege bzw. Genitalien oder diese Bereiche nicht gekühlt werden sollen, da in diesen Bereichen eine Abnahme an Volumen nicht erwünscht ist wie z.B. im Brustbereich

## Patentansprüche

1. Nicht-therapeutische Verwendung eines dreidimensionalen, mit textilem oder nicht textilem Material umschlossenen Vliesmaterials (1) mit einer Dicke von 2mm-10mm, das in Verbindung mit darin eingebrachten Flüssigkeiten Körperstellen kühlt, zur Erzielung einer hautbildverbessernden, hautstraffenden, faltenmindernden, anticellulitischen und/oder fettabbauenden Wirkung, wobei das Vliesmaterial in Form von Kleidung oder in Form von an Kleidung angeordneten Pads vorliegt sowie waschbar und mehrfach wiederverwendbar ist.

2. Verwendung nach Anspruch 1, **dadurch gekennzeichnet, dass** die Fasern und/oder Strukturen des Vliesmaterials mit Superabsorber beschichtet oder anderweitig mit Superabsorber fest verbunden sind und/oder aus superabsorbierenden Fasern gebildet sind, die eine dreidimensionale Netzstruktur bilden.

3. Verwendung nach einem oder mehreren der vorhergehenden Ansprüche, **dadurch gekennzeichnet, dass** das Vliesmaterial so ausgebildet ist, dass es sich nach Einbringen von Flüssigkeit und Ausdrücken der nichtaufgenommenen Flüssigkeit und/oder durch Abtrocknen der Außenfläche trocken anfühlt und/oder dass es keine Flüssigkeit in Form von Tropfen mehr abgibt und die Flüssigkeit gebunden hält und nur durch Verdunstung wieder abgibt.

4. Verwendung nach einem oder mehreren der vorhergehenden Ansprüche, **dadurch gekennzeichnet, dass** das Vliesmaterial zum Schutz und aus Gründen der Handhabbarkeit sowie des Designs mit einer Hülle umgeben ist, die aus textilen oder nichttextilen Stoffen gebildet ist.

5. Verwendung nach einem der vorhergehenden Ansprüche, **dadurch gekennzeichnet, dass** die Kleidung aus der folgenden Gruppe ausgewählt ist: Oberteile mit oder ohne Ärmel, kurz oder langärmelig, Westen, Shirts, Jacken, Kleider, Anzüge, Overalls, Shapingwäsche- und Kleidung, Kompressionskleidung- oder Accessoires, Röcke, Unterwäsche, Hosen, Leggings, Chaps oder Strümpfe.

6. Verwendung nach einem der vorhergehenden Ansprüche, **dadurch gekennzeichnet, dass** das Vliesmaterial vor der Anwendung in einem Kühlbehälter vorgekühlt wird und/oder dass während der Anwendung zur Verstärkung der hautbildverbessernden, hautstraffenden, faltenmindernden, anticellulitischen und/oder fettabbauenden Wirkung kältere Substanzen oder Materialien an einer nach außen gewandten Seite des Vliesmaterials angeordnet werden.

7. Verwendung nach einem der vorhergehenden Ansprüche, **dadurch gekennzeichnet, dass** Messgeräte zur Überwachung der hautbildverbessernden, hautstraffenden, faltenmindernden, anticellulitischen und/oder fettabbauenden Wirkung verwendet werden, wobei die Überwachung vorzugsweise mit Hilfe von stationären oder mobilen IT-Systemen oder Kommunikationssystemen erfolgt.

## Claims

1. Nontherapeutic use of a three-dimensional nonwoven material (1) surrounded with textile or nontextile material and having a thickness of 2 mm-10 mm which, in conjunction with liquids incorporated therein, cools body areas, for achieving a skin-appearance-improving, skin-firming, wrinkle-diminishing, anticellulite and/or fat-burning effect, wherein the nonwoven material is present in the form of clothing or in the form of pads arranged on clothing, and is washable and reusable several times.

2. Use according to claim 1, **characterized in that** the fibers and/or structures of the non-woven material are coated with superabsorber or are firmly attached to superabsorber in another way and/or are formed from superabsorbent fibers which form a three-dimensional network structure.

3. Use according to one or multiple of the preceding claims, **characterized in that** the nonwoven material is configured in such a way that, after introducing liquid and squeezing out the non-absorbed liquid and/or by drying the outer surface, feels dry and/or no longer releases liquid in the form of drops and keeps the liquid in bonded form and only releases it again as a result of evaporation.

4. Use according to one or multiple of the preceding claims, **characterized in that** the nonwoven material is surrounded, for protection and for reasons of handleability and also of design, with a covering which is formed of textile or nontextile materials.

5. Use according to any one of the preceding claims, **characterized in that** the clothing is selected from the following group: tops with or without sleeves, short- or long-sleeved, vests, shirts, jackets, dresses, suits, overalls, shaping hosiery and clothing, compression clothing or accessories, skirts, underwear, trousers, leggings, chaps or stockings.

6. Use according to any one of the preceding claims, **characterized in that** the non-woven material is precooled in a cooling container before being used and/or that colder substances or materials are arranged on the side of the nonwoven material facing outwards during the application, for intensifying the skin-appearance-improving, skin-firming, wrinkle-diminishing, anticellulite and/or fat-burning effect.

7. Use according to any one of the preceding claims, **characterized in that** measuring instruments are used for monitoring the skin-appearance-improving, skin-firming, wrinkle-diminishing, anticellulite and/or fat-burning effect, wherein the monitoring is effected preferably with the help of stationary or mobile IT systems or communication systems.

## Revendications

1. Utilisation non thérapeutique d'un matériau non tissé (1) tridimensionnel enfermé dans un matériau textile ou non textile et présentant une épaisseur de 2 mm à 10 mm, qui réfrigère des parties du corps en combinaison avec des liquides introduits dans celui-ci, afin d'obtenir un effet d'amélioration de l'aspect de la peau, de raffermissement de la peau, de réduction des rides, de prévention de la cellulite et/ou d'élimination des graisses, le matériau non tissé se présentant sous la forme d'un vêtement ou sous la forme de tampons disposés sur un vêtement et étant lavable et réutilisable plusieurs fois.

2. Utilisation selon la revendication 1, **caractérisée en ce que** les fibres et/ou les structures du matériau non tissé sont revêtues de polymère superabsorbant ou sont reliées d'une autre manière à du polymère superabsorbant et/ou sont formées en fibres superabsorbantes, qui forment une structure réticulée tridimensionnelle.

3. Utilisation selon une ou plusieurs des revendications précédentes, **caractérisée en ce que** le matériau non tissé est conçu de façon à présenter un toucher sec après l'introduction de liquide et l'extraction du liquide non absorbé et/ou par le séchage de la surface extérieure et/ou **en ce qu'**il ne dégage plus de liquide sous la forme de gouttes et conserve le liquide lié et ne le restitue que par évaporation.

4. Utilisation selon une ou plusieurs des revendications précédentes, **caractérisée en ce que**, pour le protéger et pour des raisons de maniabilité et d'esthétique, le matériau non tissé est entouré d'une housse, qui est formée en matières textiles ou non textiles.

5. Utilisation selon l'une des revendications précédentes, **caractérisée en ce que** le vêtement est sélectionné dans le groupe suivant : hauts avec ou sans manches, à manches courtes ou longues, gilets, t-shirts, vestes, robes, costumes, combinaisons, lingerie et vêtements sculptants, vêtements ou accessoires de compression, jupes, sous-vêtements, pantalons, leggings, chaps ou chaussettes.

6. Utilisation selon l'une des revendications précédentes, **caractérisée en ce que** le matériau non tissé est préréfrigéré dans un contenant réfrigérant avant l'utilisation et/ou **en ce que**, pendant l'utilisation, des substances ou matériaux plus froids sont disposés sur un côté tourné vers l'extérieur du matériau non tissé pour renforcer l'effet d'amélioration de l'aspect de la peau, de raffermissement de la peau, de réduction des rides, de prévention de la cellulite et/ou d'élimination des graisses.

7. Utilisation selon l'une des revendications précédentes, **caractérisée en ce que** des appareils de mesure sont utilisés pour surveiller l'effet d'amélioration de l'aspect de la peau, de raffermissement de la peau, de réduction des rides, de prévention de la cellulite et/ou d'élimination des graisses, la surveillance étant de préférence effectuée à l'aide de systèmes de TI ou de systèmes de communication fixes ou mobiles.
